# EUROPEAN PATENT APPLICATION

(11) **EP 3 991 634 A1**
(43) Date of publication of application: **04.05.2022**
(21) Application number: 20204694.2
(22) Date of filing: 29.10.2020
(51) Int. Cl.: A61B 3/103, A61B 3/00, A61B 3/107

(54) **METHOD AND DEVICE FOR DETERMINING A REFRACTIVE ERROR**

(71) Applicant: Carl Zeiss Vision International GmbH, 73430 Aalen (DE)
(72) Inventor: Leube, Alexander, 73434 Aalen (DE); Nehrbass, Eric, 73431 Aalen (DE); Wahl, Siegfried, 73072 Donzdorf (DE)
(74) Representative: Altmann Stößel Dick Patentanwälte PartG mbB

(57) **Abstract**

The present invention relates to a method, computer program and a device (110) for determining at least one refractive error (172) of at least one eye (112) of a person using at least one mobile communication device (114). Herein, the method comprising the following steps:
a) displaying at least one illumination pattern (128) to at least one eye (112) of a person;
b) capturing at least one image (168) picturing at least one reflection 8140) of the at least one illumination pattern (128) illuminating at least one portion of the at least one eye (112) of the person; and
c) determining a value for at least one refractive error (172) of the at least one eye (112) of the person by processing the at least one image (168),
wherein the at least one mobile communication device (114) comprises a screen (118) which is configured to perform step a) and at least one camera (120) which is configured to perform step b).

The method, the computer program and the device allow determining the at least one refractive error (172) of at least one eye (112) of a person using at least one mobile communication device (114) with increased precision, accuracy, and reliability.

## Description

### Field of the invention

The present invention relates to a method, a device and a computer program for determining at least one refractive error of at least one eye of a person using at least one mobile communication device and to a related method for producing at least one spectacle lens for at least one eye of a person.

### Related art

Various methods, devices and computer programs for determining a refractive error of at least one eye of a person are known. Herein, the terms "refraction" or "refractive" refer to a bending of incident light entering the interior of the eye via the pupil. For determining a value for the refractive error of the eye subjective approaches are, typically, applied in which an optometrist or an ophthalmologist performs an interactive test with the person. Herein, symbols, in particular in form of numbers, letters, or logos, are provided on a board, such as a cardboard, in a booklet, or on a screen to the person who, consecutively, views through a plurality of optical lenses having different optical refraction until a spectacle lens is determined by which the person can best recognize the smallest symbols. Thereafter, this procedure is repeated with measuring glasses having a different cylindrical refraction. Alternatively or in addition, an objective approach can be used, in which the optometrist or the ophthalmologist uses an autorefractive device. In this manner, values for the spherical and the cylindrical proportions of the refractive error can be obtained independently, in particular by determining a curvature of the cornea of at least one eye of a person.

However, the known approaches require both the presence of an optometrist or an ophthalmologist and a satisfactory communication between the optometrist or the ophthalmologist, on one hand, and the person, on the other hand, which is not always possible, in particular due to a consequence of language differences between the person and the optometrist or the ophthalmologist, or a disease, respectively. Further, as indicated above, the sophisticated device which is, typically, used for the objective approach requires high investment, maintenance and operation expenses that cannot be afforded at every place, in particularly viewed on a global scale.

US 5,526,073 A discloses a device to enable the measurement of a tridimensional shape of a partially reflecting anatomical surface of a human body, specifically the cornea, to be spatially located. The device, which is based upon a computer and a TV camera, comprises a body made of a transparent material (PERSPEX TM) wherein a conical hole has been drilled. Circular, frusto-conical, alternatively transparent and opaque, retro-illuminated, reference lines are provided in the conical surface of the hole. Furthermore, the device comprises a photoelectrical couple (an emitter: IRED or LED; and a sensor: photodiode or phototransistor) arranged at diametrically opposed points of the external base of the cone, such that when the cone is moved close to the eye to be analyzed, the apex of the eye intercepts the sight line between the emitter and the sensor at a pre-established distance from the cone. The light patterns reflected from the reference sight lines upon the cornea, as detected from the TV camera arranged near the apex of the cone and upon being suitably processed by said computer, enable the shape of the cornea itself as well as the curvature of all points thereof within a relatively large area to be analyzed.

US 7,465,049 B2 discloses a portable ophthalmic device comprising a supporting part to which a cellular phone having a photographing camera is mounted detachably and a main body which is provided with the supporting part integrally and having an illumination optical system projecting an illumination beam toward photographing objective eyes along an illumination optical axis intersected at a predetermined angle with a photographing optical axis.

US 9,833,140 B2 discloses portable corneal topographers and portable corneal topographer modules. Herein, a corneal topographer module comprises a housing having a first aperture and a second aperture formed therethrough; and a plurality of optical components disposed within the housing. The plurality of optical components are arranged to direct light generated by a light source through the first aperture to the cornea via a first optical channel when a cornea of a patient's eye is located adjacent to the first aperture; and direct reflected light from the cornea into the housing through the first aperture and to a light detector of a mobile device via a second optical channel when the corneal topographer module is mechanically coupled to the mobile device.

US 9,839,352 B2 discloses a device for enabling corneal topography including an attachment to align a placido disc illumination system with a camera of a mobile communication device. The placido disc illumination system generates concentric rings and reflects the concentric rings off a cornea. A portion of the reflected concentric rings are utilized to confirm vertex distance. The device further comprises a memory, a processor, and computer-readable instructions in a mobile communication device. The camera captures an image of reflected concentric rings and communicates the captured image of the reflected concentric rings to an external computing device. A method for performing corneal topography utilizes a mobile computing and/or communication device, projects a plurality of peripheral concentric rings onto a subject's cornea and projects center rings onto the subject's cornea. The method further includes capturing, via a smartphone camera, an image of the projected peripheral concentric rings and the center rings.

US 9,854,965 B2 discloses a portable device with a specially configured targeting light source that aligns the eye, mitigates accommodation, and provides accurate results. Unlike stationary, closed view autorefractors, this device typically is portable, self-usable, relatively inexpensive, enabling more widespread use across the world.

DE 10 2018 109 726 A1 discloses a device for measuring the cornea of a test person, the device comprising: an image acquisition device arranged to acquire image data of an iris of the person from a plurality of viewpoints through imaging ray paths which pass through the cornea; and a computer unit, the computer unit being arranged to: providing a mathematical model of an anterior segment of the subject's eye with a mathematical model of the cornea and the iris; identifying and registering image features of the iris that are present in multiple images of the image data; determining deviations between actual positions of the image features of the iris in the images acquired from multiple viewpoints and expected positions of the image features of the iris in the images acquired from multiple viewpoints, taking into account the mathematical model of the cornea and the location of the iris; adapting parameters of the mathematical model of the cornea such that the deviations are minimized; and determining a measure of the cornea from the adapted mathematical model of the cornea.

WO 2019/165227 A1 discloses a mobile communication device-based corneal topography system which includes an illumination system, a mobile communication device and a corneal topography optical housing. The illumination system is configured to generate an illumination pattern and to generate reflections of the illumination pattern off a cornea of a subject, wherein the illumination system is aligned along an axis of centers of the illumination pattern. The mobile communication device includes an image sensor to capture an image of the reflected illumination pattern. The corneal topography optical housing is coupled to the illumination system and the mobile communication device, wherein the corneal topography optical housing supports and aligns the illumination system with the image sensor of the mobile communication device. The corneal topography optical housing includes an imaging system coupled to the image sensor.

G.P. Butel, G.A. Smith, and J.H. Burge, Deflectometry using portable devices, Optical Engineering 54(2), 025111, 2015, describes that deflectometry is a powerful metrology technique that uses off-the-shelf equipment to achieve nanometer-level accuracy surface measurements. However, there is no portable device to quickly measure eyeglasses, lenses, or mirrors. They present an entirely portable new deflectometry technique that runs on any Android^{™} smartphone with a front-facing camera, thereby overcoming some specific issues of portable devices like screen nonlinearity and automatic gain control. They demonstrate their application by measuring an amateur telescope mirror and simulating a measurement of the faulty Hubble Space Telescope primary mirror. Their technique can, in less than 1 min, measure surface errors with accuracy up to 50 nm RMS, simply using a smartphone.

Z. Zhang, Y. Wang, S. Huang, Y. Liu, C. Chang, F. Gao, and X. Jiang, Three-Dimensional Shape Measurements of Specular Objects Using Phase-Measuring Deflectometry, Review, Sensors 2017, 17, 2835, describe that the fast development in the fields of integrated circuits, photovoltaics, the automobile industry, advanced manufacturing, and astronomy have led to the importance and necessity of quickly and accurately obtaining three-dimensional (3D) shape data of specular surfaces for quality control and function evaluation. Owing to the advantages of a large dynamic range, non-contact operation, full-field and fast acquisition, high accuracy, and automatic data processing, phase-measuring deflectometry (PMD, also called fringe reflection profilometry) has been widely studied and applied in many fields. Phase information coded in the reflected fringe patterns relates to the local slope and height of the measured specular objects. The 3D shape is obtained by integrating the local gradient data or directly calculating the depth data from the phase information. They present a review of the relevant techniques regarding classical PMD. The improved PMD technique is then used to measure specular objects having discontinuous and/or isolated surfaces. Some influential factors on the measured results are presented.

F. Willomitzer, C.-K. Yeh, V. Gupta, W. Spies, F. Schiffers, M. Walton, O. Cossairt, *Uncalibrated Deflectometry with a Mobile Device on Extended Specular Surfaces,* introduce a system and methods for a three-dimensional measurement of extended specular surfaces with high surface normal variations. The system consists only of a mobile hand-held device and exploits screen and front camera for deflectometry-based surface measurements. They demonstrate high quality measurements without the need for an offline calibration procedure. In addition, they develop a multi-view technique to compensate for the small screen of a mobile device so that large surfaces can be densely reconstructed in their entirety. This work is a first step towards developing a self-calibrating deflectometry procedure capable of taking 3D surface measurements of specular objects in the wild and accessible to persons with little to no technical imaging experience.

### Problem to be solved

In particular with respect to the disclosure of any one of US 7,465,049 B2, US 9,833,140 B2, US 9,839,352 B2, or WO 2019/165227 A1, it is therefore an objective of the present invention to provide a method, a device and a computer program for determining a refractive error of at least one eye of a person using a mobile communication device and a related method for producing at least one spectacle lens for the at least one eye of the person, which at least partially overcome the above-mentioned problems of the state of the art.

It is a particular objective of the present invention to be able to determine values for the spherical and the cylindrical proportions of the refractive error by applying a simple and easy-to-use approach. Thereby, it is desirable to be able to determine the desired values without requiring an optometrist, an ophthalmologist, a set of measuring glasses and/or a sophisticated device, such as an autorefractive device, and/or an additional attachment to a smartphone designated for this purpose. In particular, it is desirable to determine the refractive error of at least one eye of the person in a fashion which can be applied on a global scale to all kinds of persons, whereby difficulties with communication or compliance can be avoided as far as possible.

### Summary of the invention

This problem is solved by a method, a device and a computer program for determining a refractive error of at least one eye of a person using at least one mobile communication device and a related method for producing at least one spectacle lens for the at least one eye of the person with the features of the independent claims. Preferred embodiments, which might be realized in an isolated fashion or in any arbitrary combination, are listed in the dependent claims.

As used in the following, the terms "have", "comprise" or "include" or any arbitrary grammatical variations thereof are used in a non-exclusive way. Thus, these terms may refer to both a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, as used in the following, the terms "preferably", "more preferably", "particularly", "more particularly", or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in this way with other features of the invention.

In a first aspect, the present invention relates to a method for determining at least one refractive error of at least one eye of a person using at least one mobile communication device. The method according to the present invention comprises the following steps a) to c) which may, preferably, be performed in the indicated order starting with step a) and continuing with step b) and, subsequently, with step c); however, a different order may be possible at least in part. Further, additional method steps might be provided which may be indicated herein or not. Further, two or all of the method steps might be performed simultaneously, at least partially. Further, at least one of the method steps might be performed twice or more than twice, in a repeated fashion.

The method for determining at least one refractive error of at least one eye of a person using at least one mobile communication device comprises the following steps:
a) displaying at least one illumination pattern to at least one eye of a person;
b) capturing at least one image picturing at least one reflection of the at least one illumination pattern illuminating at least one portion of the at least one eye, preferably of at least one portion of a cornea, of the person; and
c) determining a value for at least one refractive error of the at least one eye of the person by processing the at least one image,
wherein the at least one mobile communication device comprises a screen which is configured to perform step a) and at least one camera which is configured to perform step b).

As indicated above, the terms "refraction" or "refractive" refer to a bending of incident light entering an interior of the eye of a person via the pupil. Instead of the term "person", a different term, such as "subject", "test person", "user", or "wearer of eye glasses", may also be applicable. Herein, the method can be used for individually determining the refractive error of each eye of a person in a consecutive or in a simultaneous fashion.

The present method for determining at least one refractive error of at least one eye of a person using at least one mobile communication device can, preferably, be used in a method for producing at least one spectacle lens for the at least one eye of the person as described below in more detail. Based on standard ISO 13666:2019(E), also referred to herein as the "standard", Section 3.5.2, the term "spectacle lens" relates to an optical lens which is used within the framework of the present invention for determining and/or correcting a defective vision of a person, wherein the optical lens is carried in front of the eye of the person, thereby avoiding a direct contact with the eye.

In particular, the determining of the at least one refractive error of at least one eye of a person can comprise determining a spherocylindrical lens which is, in general, used as a spectacle lens to correct the at least one refractive error of the at least one eye of the person. For describing the spherocylindrical lens, various approaches are possible. As defined in the standard, Section 3.6.6, the term "spherocylindrical lens" refers to a spectacle lens having a spherical surface and a cylindrical surface. Further, the spherocylindrical lens is defined, according to Section 3.13.1, as a spectacle lens which combines a paraxial, parallel beam of light in two individual, mutually perpendicular focal lines, whereby the spectacle lens has an apex refractive power only in the two main sections. Further, the term "apex refractive power" is, according to Section 3.10.7, defined as a reciprocal value of the width of the paraxial section. As further defined in Section 3.13.2, the term "main section" relates to one of two perpendicular meridians of the spectacle lens having an astigmatic effect being parallel to the two focal lines. Herein, the term "astigmatic effect" corresponds to an "astigmatic difference" which is defined in Section 3.13.6 as a difference between the value of the apex refractive power in the second main section and the value of the apex refractive power in the first main section. Further, the "cylindrical power" refers, according to Section 3.13.7, to an algebraic difference between the refractive values of the main sections, wherein the refractive value of a particular main section being used as a reference is subtracted from the refractive value of the other main section, while the "cylinder axis" indicates according to Section 3.13.8 the direction of the main section of the spectacle lens whose apex refractive index is used as the reference.

As an alternative, L. N. Thibos, W. Wheeler und D. Horner (1997), Power Vectors: An Application of Fourier Analysis to the Description and Statistical Analysis of Refractive Error, Optometry and Vision Science 74 (6), S. 367-375, propose to approach the description of a spherocylindrical lens from a viewpoint of Fourier analysis of a power profile. They show that the familiar sine-squared law leads naturally to a Fourier series representation with exactly three Fourier coefficients, representing natural parameters of a thin lens. Herein, a constant term corresponds to a mean spherical equivalent (MSE) power, whereas amplitude and phase of the harmonic correspond to the power and axis of a Jackson cross-cylinder (JCC) lens, respectively. Expressing the Fourier series in rectangular form leads to the representation of an arbitrary spherocylindrical lens as sum of a spherical lens and two cross-cylinders, one at axis 0° and the other at axis 45°. The power of these three component lenses may be interpreted as (x, y, z) coordinates of a vector representation of the power profile. The power vector representation of a spherocylindrical lens can be used for numerical and graphical analysis of optometric data for problems involving lens combinations, comparison of different lenses, and statistical distribution of refractive errors.

The method according to the present invention is a computer-implemented method. As used herein, the term "computer-implemented method" refers to a method which involves at least one mobile communication device which functions as a programmable device, wherein the at least one mobile communication device may, in particular, comprise at last one evaluation unit, wherein at least one of the features of the method is performed by using at least one computer program. In accordance with the present invention, the computer program may be provided on the at least one mobile communication device, or the at least one mobile communication device may have access to the computer program via a network, such as an in-house network or the internet.

As used herein, the term "mobile communication device" refers to at least one of a smartphone, a tablet, or a personal digital assistant, which can be carried by the person and, may thus, move together with the person. In general, the at least one mobile communication device comprises at least one screen, at least one camera, and at least one processing unit, wherein a mobile operating system running on the processing unit may be configured to facilitate a use of software, internet, and multimedia functionalities, in particular by using at least one wireless communications protocol, such as Wi-Fi or Bluetooth. Further, the at least one processing unit may comprise the at last one evaluation unit. Further, the at least one mobile communication device may comprise at least one sensor which may, in particular, be selected from at least one of a gyroscope, an accelerometer, a proximity sensor, a magnetometer, or a barometer. However, further kinds of sensors may also be conceivable.

According to step a), at least one illumination pattern may be displayed to at least one eye of a person. As used herein, the term "illumination pattern" refers to an arbitrary graphic structure which can be provided by the at least one screen of the at least one mobile communication device in a fashion that it illuminates at least one eye of a person, preferably at least one portion of the cornea thereof. Herein, the illumination pattern can be provided in one or more colors, or, alternatively or in addition, in form of an infrared pattern. In a preferred embodiment, the illumination pattern may comprise at least one wavelength or wavelength range which can be provided by the at least one screen of the at least one mobile communication device according to step a) and which can, further, be captured by the at least one camera of the at least one mobile communication according to step b).

In accordance with the present invention, the at least one screen of the at least one mobile communication device is used for performing step a). In a particularly preferred embodiment, the at least one screen may be located in front of the at least one mobile communication device, wherein the at least one mobile communication device can be arranged in a fashion that the at least one screen may face the at least one eye of the person, preferably at least one portion of the cornea thereof. However, other arrangements in which the at least one mobile communication device may comprise at least one additional screen which may, alternatively or in addition, be used for this purpose, in particular by using at least one additional optical element, such as at least one optical mirror, may also be conceivable the person skilled in the art. In a further arrangement, the screens of more than one mobile communication device can be used in a consecutive or, preferably, a simultaneous fashion.

In a preferred embodiment, the illumination pattern may be a periodic illumination pattern. As generally used, the term "periodic illumination pattern" refers to a particular pattern which comprises a spatially oriented period in one, two or three dimensions within which the structure of the periodic illumination pattern is repeated. In the periodic illumination pattern, the structure of the periodic pattern can be displayed in a repeated fashion, whereby similar points or areas may be generated over the structure of the pattern by repetition. Herein, similar points or areas may, preferably, be provided as periodic maxima or minima of the periodic illumination pattern, such as a stripe pattern or a dot pattern. In particular, the periodic illumination pattern can be represented by at least one periodic function. Herein, the term "periodic function" refers to an instruction comprising at least one specification and at least one parameter which are jointly configured to provide a temporally or, preferably, spatially repeated alteration of the periodic illumination pattern. In particular, the periodic function can be selected from a sine function, a cosine function or a superposition thereof. However, other periodic functions are also conceivable. Preferably, the periodic illumination pattern may comprise at least one spatial frequency. As generally used, the term "spatial frequency" denotes an inverse value of a spatial distance between two adjacent similar points, in particular a maximum or a minimum, within the spatially periodic alteration of the periodic illumination pattern, wherein the spatial distance may be specified in a unit of 1/m or, in particular if a distance from the at least one eye of the person, preferably of at least one portion of the cornea thereof, is known, alternatively or in addition, as a dimensionless number, for example per degree or per cycle. However, other ways of determining the spatial frequency from the pattern are conceivable for the person skilled in the art, for example from a distance of points of equal intensity.

In a further preferred embodiment, the illumination pattern may be or comprise a symbol. As generally used, the term "symbol" refers to a two-dimensional representation of at least one of an object or an abstract idea, such as a number, a letter, a logo, or a geometric figure, which can be modified in an arbitrary fashion. By way of example, the geometric figure may be selected from a cross, a circle, or a rectangle, such as a square. However, other kinds of geometric figures may also be conceivable.

In a further preferred embodiment, the illumination pattern may be or comprise a random pattern. As used herein, the term "random pattern" refers to a particular kind of illumination pattern which is generated by the at least one processing unit of the at least one mobile communication device by using a random pattern generator to be displayed by the at least one screen of the at least one mobile communication device.

In a particularly preferred embodiment, the at least one mobile communication device may, independent from a shape or form of the at least one illumination pattern, configured to have access to the at least one illumination pattern as displayed during step a) or to a reference thereto. As used herein, the term "access" refers to a configuration of the at least one mobile communication device which enables it to receive the at least one illumination pattern that has been displayed during step a) or a piece of information which is configured to recover the at least one illumination pattern as displayed during step a) upon request. For this purpose, the at least one illumination pattern may be stored, preferably in at last one of a pixel-wise manner or in a compressed fashion, in at least one storage unit as further comprised by the at least one mobile communication device or attached to the at least one mobile communication device. The at least one piece of information may be or comprise at least one specification which is configured to recover the at least one illumination pattern. By way of example, the at least one piece of information may be selected from at least one specification and at least one parameter of at least one periodic function. Herein, the specification may, in particular, provide the type of periodic function, such a sine or a cosine function, while the parameter may be a parameter of the periodic function, such as a value for a spatial frequency.

As an alternative, a reference to the at least one illumination pattern that has been displayed during step a) may be stored in the at least one storage unit as comprised by the at least one mobile communication device or attached to the at least one mobile communication device. As used herein, the term "reference" refers to at least one piece of information which allows providing the at least one illumination pattern upon demand as expressed by a computer program which runs on at least one of the at least one mobile communication device or on at least one server communicating with the mobile communication device. Herein, the at least one illumination pattern may be stored on the at least one mobile communication device or on the at least one server. Further, the at least one piece of information which provides access to the at least one illumination pattern may be at least one code which may be assigned to the at least one illumination pattern for reference, such as a numeric or an alphanumeric value assigned to the at least one illumination pattern, preferably in a unique fashion.

According to step b), at least one image which pictures at least one reflection of the at least one illumination pattern illuminating at least one portion of the at least one eye of the person, preferably at least one portion of the cornea thereof, is captured. As used herein, the term "image" refers to a two-dimensional representation or to a three-dimensional representation of the at least one reflection of the at least one illumination pattern which illuminates at least one portion of the at least one eye of the person, preferably at least one portion of the cornea thereof. Further, the term "representation" refers to a set of data captured by using at least one optical recording unit, hereinafter referred to as at least one "camera". As further used herein, the term "capturing" or any grammatical variation thereof refers to recording a single image or a plurality of images, in particular in form of a video sequence. As further used herein, the term "picturing" or any grammatical variation thereof describes that the at least one image comprises a picture of the at least one reflection of the at least one illumination pattern which illuminates the at least one portion of the at least one eye of the person, preferably at least one portion of the cornea thereof.

In accordance with the present invention, the at least one camera of the at least one mobile communication device is used for performing step b). In particular, the at least one camera may be at least one of a front camera or a rear camera as comprised by the at least one mobile communication device. In a particularly preferred embodiment, the at least one front camera as located in front of the at least one mobile communication device may be used for this purpose, wherein the at least one mobile communication device can be arranged in a fashion that the at least one front camera may face the at least one eye of the person, preferably at least one portion of the cornea thereof. However, other arrangements in which the at least one rear camera of the at least one mobile communication device may, alternatively or in addition, be used for this purpose, in particular by using at least one additional optical element, such as at least one optical mirror, may also be conceivable the person skilled in the art. In a further arrangement, the cameras of more than one mobile communication device can be used in a consecutive or, preferably, a simultaneous fashion.

According to step c), a value for at least one refractive error of the at least one eye of the person is determined by processing the at least one image which has been captured during step b). As generally used, the term "determining" or any grammatical variation thereof refers to a process of generating at least one representative result, such as a plurality of representative results, in particular by applying the method for determining at least one refractive error of at least one eye of a person using at least one mobile communication device according to the present invention. As further used herein, the term "processing" or any grammatical variation thereof refers to applying at least one algorithm to extract at least one piece of picture information from the at least one image. The at least one algorithm may be configured to determine the value for the at least one refractive error of the at least one eye of the person by evaluating the two-dimensional representation or the three-dimensional representation of the image according to a scheme. In particular, step c) may comprise comparing at least a portion of the at least one image with a corresponding portion of the at least one illumination pattern, especially, by at least one of in a pixel-wise fashion or in a comprehensive fashion. For the comprehensive fashion, an analysis tool well-known to the skilled person, such as a Fast Fourier Transformation (FFT) or a wavelet analysis, may be used. However, other ways of comparing the at least one image with the at least one illumination pattern may also be feasible.

The determination of the value for at least one refractive error of the at least one eye of the person may be performed in accordance with a predefined scheme, however, artificial intelligence, in particular machine learning, may also be applied, especially by using a neuronal network. As generally used, the term "machine learning" refers to a process applying artificial intelligence to automatically generate a statistical model for classification or regression. A machine learning algorithm configured to generate the desired model based on a large number of training data sets can, preferably, be used. Herein, the machine learning algorithm can be a supervised algorithm or a self-learning algorithm. The machine learning algorithm can use and/or comprise a neural network, which may, preferably, be developed into a trained neural network by using the at least one training data set. The neural network may comprise at least one element selected from hierarchical decision trees, Hough forest, regression forest, Convolutional Neural Network (CNN), Deep Neural Network (DNN) Residual Neural Network, Pixel-wise Voting, Pixel-wise Fusion Network, Deep learning. Alternatively or additionally, the use of at least one other artificial intelligence method, preferably a kernel method, especially a Support Vector Machine (SVM), may also be possible. From the at least one image as captured according to step b), the desired value for the at least one refractive error of the at least one eye of the person can be determined by image processing.

According to the present invention, the at least one screen and the at least one camera as comprised by the at least one mobile communication device is used for the purposes of the present invention in a fashion that the at least one screen is configured to perform step a) and that the at least one camera is configured to perform step b). With respect to step c), reference can be made to the description below. This kind of use of the mobile communication device for determining of at least one refractive error of at least one eye of a person is, thus, in particular contrast to a corresponding determination according to the prior art, especially with respect to the disclosure of US 7,465,049 B2, US 9,833,140 B2, US 9,839,352 B2, or WO 2019/165227 A1 as referred to above, each of which requires a separate illumination device which is configured to display at least one illumination pattern to the at least one eye of the person in accordance with step a).

In a particularly preferred embodiment, the at least one mobile communication device may, in addition, be configured to perform step c). For this purpose, the at least one mobile communication device may comprise at least one evaluation unit, wherein the at least one evaluation unit may be configured to determine the value for the at least one refractive error of the at least one eye of the person by processing the at least one image. Alternatively or in addition, the at least one mobile communication device may be configured to perform step c) by using at least one communication interface as further comprised by the at least one mobile communication device. In this embodiment, the at least one communication interface may be configured to exchange data with at least one server, wherein the at least one server, which is not comprised by the at least one mobile communication device, is configured to determine the value for the at least one refractive error of the at least one eye of the person by processing the at least one image and to, preferably, provide the value to the evaluation unit of the at least one mobile communication device.

As generally used, the term "communication interface" refers a transmission channel which is designated for a transmission of data. Herein, the communication interface may be arranged as a unidirectional interface which is configured to forward at least one piece of data into a single direction, either from the at least one evaluation unit to the server, or from the server to the at least one evaluation unit. Alternatively, the communication interface may be arranged as a bidirectional interface which is configured to forward at least one piece of data into one of two directions, from the at least one evaluation unit to the server, or vice versa. Thus, a particular bidirectional interface can, as an alternative, be replaced by two individual unidirectional interfaces which are configured for data transmission in an opposite direction with respect to each other. For a purpose of data transmission, the communication interface may, preferably, comprise a wireless element, which may, by way of example, operate by using at least one wireless communications protocol, such as Wi-Fi or Bluetooth. However, further kinds of communication interfaces may also be feasible.

As further generally used, the term "server" refers to a device which is configured to provide resources to a further device, typically denoted as "client", wherein the "resources", in particular, comprise at least one of computing power, such as for running at least one computer program; or data storage capacity, such as for storing at least one piece of data. By way example, a client can run a single computer program or store pieces of data distributed across multiple servers, while a single server can serve multiple clients with regard to at least one of program execution and storage requirement. While the term "server" refers to a device that is arranged within a local network, the term "cloud server" relates to a kind of server that is accessible on demand by the client via internet. As a result, neither a location of the cloud server nor a direct active management of the cloud server is accessible to the client.

In a particularly preferred embodiment, the at least one mobile communication device may, in addition, be configured to synchronize the displaying of the at least one illumination pattern according to step a) with the capturing of the at least one image according to step b). This embodiment, particularly, allows generating a definite relationship between the at least one illumination pattern as displayed according to step a) and the at least one image as captured according to step b). As a result, each feature as recognized during the processing the at least one image according to step c) can be assigned to a corresponding feature in the at least one illumination pattern according to step a) in a definite fashion.

Moreover, by providing access to the at least one illumination pattern as displayed during step a) to the at least one mobile communication device as described above or below in more detail, the determination of the value for the at least one refractive error of the at least one eye of the person, in particular by comparing the at least one image with the at least one illumination pattern, during step c) can, advantageously, be performed without requiring any assumptions, approximations or calibrations compared to the methods as used in the prior art. As a result, the determining of a value for the at least one refractive error of the at least one eye of the person according to the present invention can be performed with increased precision, accuracy, and reliability in comparison with the methods as used in the prior art.

In a particularly preferred embodiment, the processing of the at least one image according to step c) may comprise determining at least one topographical value of a surface of at least one portion of the at least one eye of the person, preferably of at least one portion of the cornea thereof. For this purpose, at least one radius of the surface of the at least one portion of the at least one eye of the person, preferably of at least one portion of the cornea thereof, may be determined. However, using a different approach for evaluating the surface may also be feasible.

In a particularly preferred embodiment, the determining of the value for the at least one refractive error of the at least one eye of the person may comprise determining at least one focal length of the at least one eye of the person. For this purpose, the at least one radius of the surface of the at least one portion of the at least one eye of the person, preferably of at least one portion of the cornea thereof, and/or at least one value for a refractive index of the at least one eye of the person, preferably of at least one portion of the cornea thereof, may be used. However, using a different geometrical property of the surface may also be feasible.

In a particular embodiment, the determining of the value for the at least one refractive error of the at least one eye of the person may, further, comprise determining at least one value for an astigmatism of the at least one eye of the person. For this purpose, at least two individual radii of the surface of the at least one portion of the at least one eye of the person, preferably of at least one portion of the cornea thereof, may be determined. In particular, at least two individual astigmatic power vectors from the at least two individual radii of the surface of the at least one portion of the at least one eye of the person, preferably of at least one portion of the cornea thereof, may be used for this purpose. Alternatively or in addition, an orientation of at least one principle meridian which can be determined from the power vectors or vice-versa, can also be used for this purpose. However, using a different approach as known by the person skilled in the art may also be feasible.

In a further aspect, the present invention refers to a computer program which comprises instructions which, when the program is executed by a computer, cause the computer to carry out the method for determining at least one refractive error of at least one eye of a person using at least one mobile communication device according to the present invention. For this purpose, a computer program may comprise instructions which can be provided by means of a computer program code which are capable of performing any or all of the steps of the methods as described elsewhere herein and, thus, to establish determining the refractive error of the at least one eye of a person when implemented on a data processing unit as comprised by the at least one mobile communication device.

In a further aspect, the present invention relates to a method for producing at least one spectacle lens. As generally, the producing of the at least one spectacle lens comprises processing at least one lens blank by adjusting at least one optical parameter of the at least one spectacle lens, wherein the processing of the lens blank is based on instructions which are configured to compensate the at least one refractive error of the at least one eye of a person as described in the prior art. According to the present invention, the determining of the refractive error of the at least one eye of the person comprises applying the method for determining at least one refractive error of at least one eye of a person using at least one mobile communication device according to the present invention.

For further details concerning the computer program or the method for producing at least one spectacle lens, reference may be made to the method for determining at least one refractive error of at least one eye of a person using at least one mobile communication device as disclosed elsewhere herein.

In a further aspect, the present invention relates to a device for determining at least one refractive error of at least one eye of a person using at least one mobile communication device. According to the present invention, the device comprises:
- at least one screen, wherein the at least one screen is configured to display at least one illumination pattern to at least one eye of a person;
- at least one camera, wherein the at least one camera is configured to capture at least one image picturing at least one reflection of the at least one illumination pattern illuminating at least one portion of the at least one eye of the person; and
- at least one evaluation unit, wherein the at least one evaluation unit is configured to determine a value for the at least one refractive error of the at least one eye of the person by processing the at least one image, or wherein the at least one evaluation unit comprises at least one communication interface configured to exchange data with at least one server, wherein the at least one server is configured to determine the value for the at least one refractive error of the at least one eye of the person by processing the at least one image,
wherein the at least one mobile communication device comprises the at least one screen and the at least one camera.

For the term "mobile communication device", reference can be made to the definition above.

In a preferred embodiment, the system may, further comprise, at least one of:
- a distance meter, wherein the distance meter is configured to determine at least one distance;
- a gyro sensor, wherein the gyro sensor is configured to determine at least one orientation of the at least one mobile communication device in space;
- a holding unit attached to the at least one mobile communication device, wherein the holding unit is configured to maintain a position of the at least one mobile communication device.

As indicated above, the at least one camera configured to capture the at least one image may, specifically, be selected from at least one of a front camera or a rear camera as comprised by the at least one mobile communication device, wherein the at least one front camera may, especially, be preferred.

Further, the at least one camera and the at least one evaluation unit of the at least one mobile communication device can, jointly, be used as the distance meter by being configured to determine the at least one distance between the at least one camera and the at least one eye of the person, preferably at least one portion of the cornea thereof.

Alternatively or in addition, further embodiments with respect to the device according to the present invention are conceivable.

For further details concerning the device for determining at least one refractive error of at least one eye of a person using at least one mobile communication device, reference may be made to the method device for determining at least one refractive error of at least one eye of a person using at least one mobile communication device as disclosed elsewhere herein.

As a particular advantage of the present invention, the methods, the computer program and the device as disclosed herein allow determining the at least one refractive error of at least one eye of a person using at least one mobile communication device with increased precision, accuracy, and reliability in comparison with the methods, computer programs and devices as used in the prior art. In particular, synchronizing the displaying of the at least one illumination pattern with the capturing of the at least one image allows generating a definite relationship between the at least one actually displayed illumination pattern and the at least one actually captured image, whereby, each feature as recognized during the processing the at least one actually captured image can be assigned to a corresponding feature in the at least one actually displayed illumination pattern in a definite fashion. Moreover, by providing access to the at least one actually displayed illumination pattern to the at least one mobile communication device, specifically to the at let one evaluation unit, determining the value for the at least one refractive error of the at least one eye of the person, in particular by comparing the at least one image with the at least one illumination pattern, can, advantageously, be performed without requiring any assumptions or approximations compared to the methods as used in the prior art.

Further, initially determining at least one value for a curvature of the cornea or at least one portion thereof of at least one eye of a person as disclosed herein in more detail can be used as a reliable starting point for determining the at least one refractive error of the at least one eye of the person, in particular, by using a known relationship between the at least one refractive error of the at least one eye of the person and at least one radius of the surface of at least one portion of the cornea of at least one eye of the person, which corresponds to an aberration of the cornea of at least one eye of the person.

In a further embodiment of the present invention, possible changes of the at least one radius of the surface of at least one portion of the cornea of at least one eye of the person can measured at least twice, preferably, multiple times. Herein, the respective measurement results can, in particular, be used for a purpose of at least one of screening or disease tracking. In particular, the device as disclosed herein can be used in a tele-medicine system or in relationship with a tele-medicine system.

Summarizing, the following Embodiments are particularly preferred within the scope of the present invention:
Embodiment 1. A method for determining at least one refractive error of at least one eye of a person using at least one mobile communication device, the method comprising the following steps:
   a) displaying at least one illumination pattern to at least one eye of a person;
   b) capturing at least one image picturing at least one reflection of the at least one illumination pattern illuminating at least one portion of the at least one eye of the person; and
   c) determining a value for at least one refractive error of the at least one eye of the person by processing the at least one image,
   wherein the at least one mobile communication device comprises a screen which is configured to perform step a) and at least one camera which is configured to perform step b).
Embodiment 2. The method according to the preceding Embodiment, wherein the at least one mobile communication device is further configured to synchronize the displaying of the at least one illumination pattern according to step a) with the capturing of the at least one image according to step b).
Embodiment 3. The method according to any one of the preceding Embodiments, wherein the at least one mobile communication device has access to the at least one illumination pattern as displayed during step a) or to a reference thereto.
Embodiment 4. The method according to any one of the preceding Embodiments, wherein step c) comprises comparing the at least one image with the at least one illumination pattern.
Embodiment 5. The method according to any one of the preceding Embodiments, wherein the at least one mobile communication device further comprises at least one evaluation unit.
Embodiment 6. The method according to the preceding Embodiment, wherein the at least one evaluation unit is configured to perform step c).
Embodiment 7. The method according to any one of the two preceding Embodiments, wherein the at least one evaluation unit comprises at least one communication interface configured to exchange data with at least one server, wherein the at least one server is configured to perform step c).
Embodiment 8. The method according to any one of the preceding Embodiments, wherein processing the at least one image comprises determining at least one topographical value of a surface of at least one portion of the at least one eye of the person.
Embodiment 9. The method according to the preceding Embodiment, wherein determining the at least one topographical value of the surface of the at least one portion of the at least one eye of the person comprises determining at least one radius of the surface of the at least one portion of the at least one eye of the person.
Embodiment 10. The method according to any one of the preceding Embodiments, wherein determining the value for the at least one refractive error of the at least one eye of the person comprises determining at least one focal length of the at least one eye of the person.
Embodiment 11. The method according to the preceding Embodiment, wherein determining the at least one focal length of the at least one eye of the person comprises using at least one of the at least one radius of the surface or at least one refractive index of the at least one portion of the at least one eye of the person.
Embodiment 12. The method according to any one of the preceding Embodiments, wherein determining the value for the at least one refractive error of the at least one eye of the person further comprises determining at least one value for an astigmatism of the at least one eye of the person.
Embodiment 13. The method according to the preceding Embodiment, wherein determining the at least one value for the astigmatism of the at least one eye of the person comprises determining at least two individual radii of the surface of the at least one portion of the at least one eye of the person.
Embodiment 14. The method according to the preceding Embodiment, wherein determining the at least one value for the astigmatism of the at least one eye of the person comprises determining at least two individual astigmatic power vectors from the at least two individual radii of the surface of the at least one portion of the at least one eye of the person.
Embodiment 15. A computer program comprising instructions which, when the program is executed by a computer, cause the computer to carry out a method for determining at least one refractive error of at least one eye of a person using at least one mobile communication device according to any one of the preceding Embodiments.
Embodiment 16. A method for producing at least spectacle lens, wherein the producing of the at least one spectacle lens comprises processing at least one lens blank by adjusting at least one optical parameter of the at least one spectacle lens, wherein the processing of the lens blank is based on instructions configured to compensate at least one refractive error of the at least one eye of a person, wherein the determining of the refractive error of the at least one eye of the person comprises using at least one mobile communication device according to any one of the preceding Embodiments.
Embodiment 17. A device for determining for determining at least one refractive error of at least one eye of a person using at least one mobile communication device, the device comprising:
   - at least one screen, wherein the at least one screen is configured to display at least one illumination pattern to at least one eye of a person;
   - at least one camera, wherein the at least one camera is configured to capture at least one image picturing at least one reflection of the at least one illumination pattern illuminating at least one portion of the at least one eye of the person; and
   - at least one evaluation unit, wherein the at least one evaluation unit is configured to determine a value for the at least one refractive error of the at least one eye of the person by processing the at least one image, or wherein the at least one evaluation unit comprises at least one communication interface configured to exchange data with at least one server, wherein the at least one server is configured to determine the value for the at least one refractive error of the at least one eye of the person by processing the at least one image,
   wherein the at least one mobile communication device comprises the at least one screen and the at least one camera.
Embodiment 18. The device according to the preceding Embodiment, wherein the at least one evaluation unit is further configured to synchronize the displaying of the at least one illumination pattern with the capturing of the at least one image.
Embodiment 19. The device according to any one of the preceding Embodiments referring to a device, wherein the evaluation unit is further configured to have access to the at least one illumination pattern as displayed by the at least one screen or to a reference thereto.
Embodiment 20. The method according to any one of the preceding Embodiments referring to a device, wherein the at least one evaluation unit is further configured to compare the at least one image with the at least one illumination pattern.
Embodiment 21. The device according to any one of the preceding Embodiments referring to a device, wherein the at least one mobile communication device further comprises at least one distance meter, wherein the at least one distance meter is configured to determine at least one distance.
Embodiment 22. The device according to any one of the preceding Embodiments referring to a device, wherein the at least one mobile communication device further comprises at least one gyro sensor, wherein the at least one gyro sensor is configured to determine at least one orientation of the at least one mobile communication device in space.
Embodiment 23. The device according to any one of the preceding Embodiments referring to a device, wherein the at least one mobile communication device further comprises at least one holding unit attached to the at least one mobile communication device, wherein the at least one holding unit is configured to maintain a position of the at least one mobile communication device.

### Short description of the Figures

Further optional features and embodiments of the present invention are disclosed in more detail in the subsequent description of preferred embodiments, preferably in conjunction with the dependent claims. Therein, the respective optional features may be realized in an isolated fashion as well as in any arbitrary feasible combination, as the skilled person will realize. It is emphasized here that the scope of the invention is not restricted by the preferred embodiments.

In the Figures:
- Figure 1: illustrates a preferred embodiment of a device for determining a refractive error of an eye of a person using a mobile communication device according to the present invention; and
- Figure 2: illustrates a preferred embodiment of a method for determining a refractive error of an eye of a person using a mobile communication device according to the present invention.

### Detailed description of the embodiments

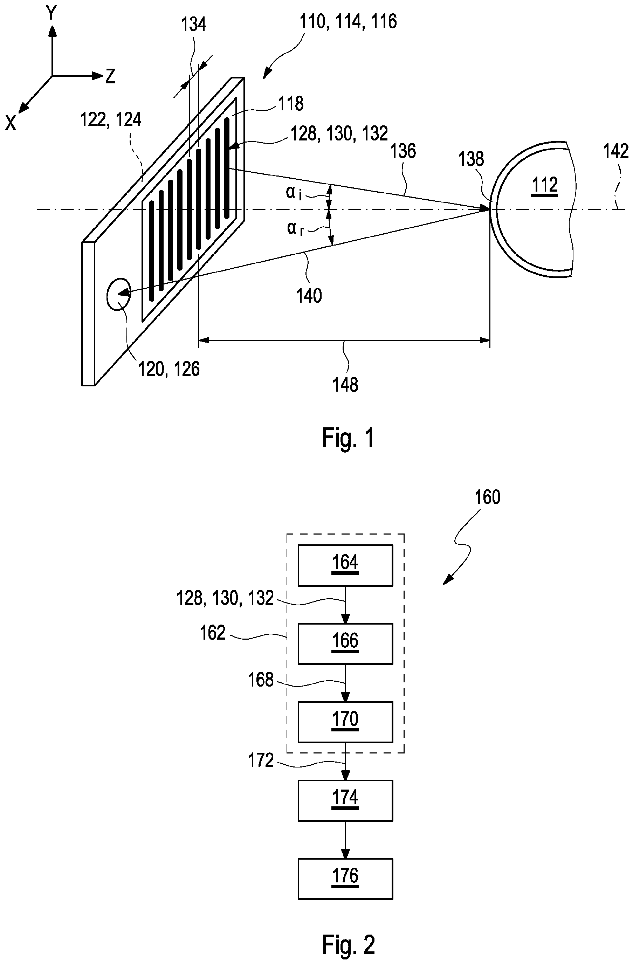

Figure 1 illustrates a preferred embodiment of a device 110 for determining a refractive error of an eye 112 of a person using a mobile communication device 114. Herein, the refractive error of both eyes 112 of the person can, preferably, be determined in a consecutive fashion. As schematically depicted in Figure 1, the exemplary device 110 of Figure 1 comprises - without limiting the scope of the invention - a smartphone 116 as a preferred example of the mobile communication device 114. Herein, the smartphone 116 has a screen 118, a camera 120, and a processing unit 122, wherein the processing unit 122may, especially, comprise an evaluation unit 124.

It is, however, emphasized that the scope of the present invention is not limited to determining the refractive error of the eye 112 by using the screen 118 located on a front of the smartphone 116; rather any other screen 118 which may, further, be comprised by the smartphone 116 can, in principle, also be used for the purposes of the present invention, especially in connection with an additional optical element, such as one or more optical mirrors (not depicted here). Similarly, the scope of the present invention is not limited to determining the refractive error of the eye 112 by using a front camera 126 of the smartphone 116; rather any other camera 120 which may, further, be comprised by the smartphone 118, such as a rear camera (not depicted here), can, in principle, also be used for the purposes of the present invention, especially in connection with an additional optical element, such as one ore more optical mirrors (not depicted here). Further, more than one smartphone 116 or other mobile communication device 114 may be used for the purposes of the present invention.

Further, a holding unit (not depicted here) may be configured to maintain the smartphone 116 in a desired position, in particular, to increase precision accuracy, and reliability of the determination of the refractive error of the eye 112.

As further illustrated in Figure 1, the screen 118 is configured to display one or more illumination patterns 128 to the eye 112. Herein, the illumination pattern 128 is a periodic illumination pattern 130 which comprises in the exemplary illustration of Figure 1 a spatially oriented period in one dimension within which the structure of the periodic illumination pattern 130 is repeated. However, repeating the periodic illumination pattern 130 in two or three dimensions may also be feasible. This particular periodic illumination pattern 130 is a stripe pattern 132 comprising a plurality of stripes which are displayed on the screen 118 in a repeated fashion, whereby similar areas are generated by repetition. In a two-dimensional repetition (not depicted here), the periodic illumination pattern 130 may comprise a plurality of dots displayed in a repeated fashion on the screen 118. Further examples are feasible. In particular, the periodic illumination pattern 130 can be represented by one or more periodic functions, preferably selected from a sine function, a cosine function or a superposition thereof. However, other periodic functions are also conceivable. As depicted here, the periodic illumination pattern 130 comprises one or more spatial frequencies each referring to an inverse value of a spatial distance 134 between two adjacent similar points, in particular a maximum or a minimum, within the spatially periodic alteration of the periodic illumination pattern 130.

As further illustrated in Figure 1, the illumination pattern 128, in particular the periodic illumination pattern 130, effects an illumination 136 of the eye 112 or a portion thereof. As a consequence of the illumination 136 of the eye 112 or the portion thereof, a proportion of the illumination 136 which impinges on the eye 112, specifically on a cornea 138 of the eye 112, is reflected by the cornea 138 of the eye 112 in form of a reflection 140 towards the smartphone 116, in particular to the front camera 126 of the smartphone 116. Hereby, a reflection angle αᵣ between an optical axis 142 of the eye 112 and a direction of the reflection 140 towards the front camera 126 of the smartphone 116, generally, differs from an illumination angle αᵢ between the optical axis 142 of the eye 112 and a direction of the illumination 136 towards the cornea 138 of the eye 112. As further depicted in Figure 1, the front camera 112 of the smartphone 116 is used here to capture at least one image which pictures a proportion of the reflection 140 of the at least one illumination pattern 128 illuminating at least one portion of the 112, specifically the cornea 138 of the eye 112.

As further illustrated in Figure 1, the smartphone 116 has a processing unit 122 which comprises here the evaluation unit 124. Again without limiting the scope of the invention, the evaluation unit 124 as comprised by the smartphone 116 is configured here to determine a value for the refractive error of the eye 112 by processing the at least one image as captured by the front camera 126. As an alternative or in addition, the evaluation unit 124 may comprise at least one communication interface (not depicted here) which may be configured to exchange data with one or more external servers (not depicted here), which are not comprised by the smartphone 116, wherein the external server is configured to determine the value for the refractive error of the eye 112 by processing the at least one image as captured by the front camera 126.

By repeating the illumination 136 of the eye 112 or the portion thereof for a plurality of points, a topographical map of a surface 144 of the eye 112, in particular of the cornea 138 of the eye 112, is generated. Herein, a radius 146 can be assigned to the surface 144 of the eye 112, in particular of the cornea 138 of the eye 112. In addition, further points in the topographical map can be obtained by using an approximation procedure, preferably standard polynomials, such as Zernike polynomials, radial polynomials, or Bessel functions, which are, generally, used for representing aberrations in topographical map. In a particular embodiment, an assignment of pixels in the illumination pattern 128 with the reflected pixels in the at least on image can be increased by individually evaluating patterns as displayed in one or more color channels on the screen. As a further advantage, a faster and more accurate measurement can be achieved in this fashion. In addition, in the stripe pattern 132 as exemplarily depicted in Figure 1, the stripe pattern 132 can, preferably, be displayed in different orientations or may have radially symmetrical stripes. Further, at least one algorithm which may, particularly, be configured to determine a three-dimensional extension of a surface of the eye 112, in particular of the cornea 138 of the eye 112, such as a SLAM algorithm, may also be used.

In particular contrast to prior art devices, such as disclosed in US 7,465,049 B2, US 9,833,140 B2, US 9,839,352 B2, or WO 2019/165227 A1 as referred to above, each of which requires an illumination device configured to display an illumination pattern to the eye 112 of the person which is separate from the smartphone 116, the device 110 for determining a refractive error of the eye 112 according to the present invention only uses the mobile communication device 114, in particular the smartphone 116, to perform both the displaying of the illumination pattern 128, in particular of the periodic illumination pattern 130, to the eye 112, in particular the cornea 138 of the eye 112, and the capturing of the at least one image which pictures a proportion of the reflection 140 of the at least one illumination pattern 128 illuminating at least one portion of the 112, specifically the cornea 138 of the eye 112. For advantages of this particular arrangement, reference may be made to the description above.

In addition, the smartphone 116 may, further, comprise at least one distance meter (not depicted here), which may be configured to determine at least one distance 148 between the eye 112, in particular the cornea 138 of the eye 112, of the person and the smartphone 116, especially the front camera 126 of the smartphone 116.

Figure 2 schematically illustrates a preferred embodiment of a method 160 for producing a spectacle lens for the at least one eye 112 of the person.

In a specifying step 162, the refractive error of the at least one eye 112 of the person is determined by using the mobile communication device 114, in particular the smartphone 116.

Herein, the specifying step 162 comprises according to step a), a displaying step 164, during which at least one illumination pattern 128, in particular the periodic illumination pattern 130, especially the stripe pattern 132 as exemplarily depicted in Figure 1, is displayed to the eye 112, in particular the cornea 138 of the eye 112, of the person.

Further, the specifying step 162 comprises according to step b), a capturing step 166, during which at least one image 168 is captured. Herein, the at least one image 168 pictures the at least one reflection 140 of the at least one illumination pattern 128 that illuminates at least one portion of the at least one eye 112, in particular the cornea 138 of the eye 112, of the person.

Further, the specifying step 162 comprises according to step c) a determining step 170, during which a value for at least one refractive error 172 of the at least one eye 112 of the person is determined by processing the at least one image 168.

For further details concerning the specifying step 162, reference can be made to the description above.

In a processing step 174, the spectacle lens is produced as well-known by the person skilled in the art by processing a lens blank (not depicted here), e.g. by milling or drilling the lens blank, based on instructions which are configured to compensate the at least one refractive error 172 of the at least one eye 112 of the person. As a result, a spectacle lens 176, which is produced in this fashion, capable of compensating the at least one refractive error 172 of the at least one eye 112 of the person.

### List of Reference Signs

- 110: device (for determining a refractive error of at least one eye of a person)
- 112: eye
- 114: mobile communication device
- 116: smartphone
- 118: screen
- 120: camera
- 122: processing unit
- 124: evaluation unit
- 126: front camera
- 128: illumination pattern
- 130: periodic illumination pattern
- 132: stripe pattern
- 134: distance
- 136: illumination
- 138: cornea
- 140: reflection
- 142: optical axis
- 144: surface
- 146: radius
- 148: distance
- 160: method (for determining a refractive error of at least one eye of a person)
- 162: specifying step
- 164: displaying step
- 166: capturing step
- 168: image
- 170: determining step
- 172: refractive error
- 174: processing step
- 176: spectacle lens

## Claims

1. A method for determining at least one refractive error (172) of at least one eye (112) of a person using at least one mobile communication device (114), the method comprising the following steps:
a) displaying at least one illumination pattern (128) to at least one eye (112) of a person;
b) capturing at least one image (168) picturing at least one reflection (140) of the at least one illumination pattern (128) illuminating at least one portion of the at least one eye (112) of the person; and
c) determining a value for at least one refractive error (172) of the at least one eye (112) of the person by processing the at least one image (168),
**characterized in**
**that** the at least one mobile communication device (114) comprises a screen (118) which is configured to perform step a) and at least one camera (120) which is configured to perform step b).

2. The method according to the preceding claim, wherein the at least one mobile communication device (114) is further configured to synchronize the displaying of the at least one illumination pattern (128) according to step a) with the capturing of the at least one image (168) according to step b).

3. The method according to any one of the preceding claims, wherein the at least one mobile communication device (114) has access to the at least one illumination pattern (128) as displayed during step a) or to a reference thereto, and wherein step c) comprises comparing the at least one image (168) with the at least one illumination pattern (128).

4. The method according to any one of the preceding claims, wherein the at least one mobile communication device (114) further comprises at least one evaluation unit (124), wherein the at least one evaluation unit (124) is at least one of: configured to perform step c), or comprising at least one communication interface configured to exchange data with at least one server, wherein the at least one server is configured to perform step c).

5. The method according to any one of the preceding claims, wherein processing the at least one image (168) comprises determining at least one topographical value of a surface (144) of at least one portion of the at least one eye (112) of the person.

6. The method according to the preceding claim, wherein determining the at least one topographical value of the surface (144) of the at least one portion of the at least one eye (112) of the person comprises determining at least one radius (146) or the at least one refractive index of the surface (144) of the at least one portion of the at least one eye (112) of the person.

7. The method according to any one of the preceding claims, wherein determining the value for the at least one refractive error (172) of the at least one eye (112) of the person comprises determining at least one focal length of the at least one eye (112) of the person.

8. The method according to the preceding claim, wherein determining the at least one focal length of the at least one eye (112) of the person comprises using at least one of the at least one radius (146) of the surface (144) or at least one refractive index of the at least one portion of the at least one eye (112) of the person.

9. The method according to any one of the preceding claims, wherein determining the value for the at least one refractive error (172) of the at least one eye (112) of the person further comprises determining at least one value for an astigmatism and its axis of the at least one eye (112) of the person.

10. A computer program comprising instructions which, when the program is executed by a computer, cause the computer to carry out a method for determining at least one refractive error (172) of at least one eye (112) of a person using at least one mobile communication device (116), the method comprising the following steps:
a) displaying at least one illumination pattern (128) to at least one eye (112) of a person;
b) capturing at least one image (168) picturing at least one reflection (140) of the at least one illumination pattern (128) illuminating at least one portion of the at least one eye (112) of the person; and
c) determining a value for at least one refractive error (172) of the at least one eye (112) of the person by processing the at least one image (168),
**characterized in**
**that** the at least one mobile communication device (114) comprises a screen (118) which is configured to perform step a) and at least one camera (120) which is configured to perform step b).

11. A method (160) for producing at least spectacle lens (176), wherein the producing of the at least one spectacle lens (176) comprises processing at least one lens blank by adjusting at least one optical parameter of the at least one spectacle lens (176), wherein the processing of the lens blank is based on instructions configured to compensate at least one refractive error (172) of the at least one eye (112) of a person, wherein the determining of the refractive error (172) of the at least one eye (112) of the person comprises using at least one mobile communication device (114) according to the following steps:
a) displaying at least one illumination pattern (128) to at least one eye (112) of a person;
b) capturing at least one image (168) picturing at least one reflection (140) of the at least one illumination pattern (128) illuminating at least one portion of the at least one eye (112) of the person; and
c) determining a value for at least one refractive error (172) of the at least one eye (112) of the person by processing the at least one image (168),
**characterized in**
**that** the at least one mobile communication device (112) comprises a screen (118) which is configured to perform step a) and at least one camera (120) which is configured to perform step b).

12. A device (110) for determining at least one refractive error (172) of at least one eye (112) of a person using at least one mobile communication device (114), the device comprising:
- at least one screen (118), wherein the at least one screen (118) is configured to display at least one illumination pattern (128) to at least one eye (112) of a person;
- at least one camera (120), wherein the at least one camera (120) is configured to capture at least one image (168) picturing at least one reflection (140) of the at least one illumination pattern (128) illuminating at least one portion of the at least one eye (112) of the person; and
- at least one evaluation unit (124), wherein the at least one evaluation unit (124) is configured to determine a value for the at least one refractive error (172) of the at least one eye (112) of the person by processing the at least one image (168), or wherein the at least one evaluation unit (124) comprises at least one communication interface configured to exchange data with at least one server, wherein the at least one server is configured to determine the value for the at least one refractive error (172) of the at least one eye (112) of the person by processing the at least one image (168),
**characterized in**
**that** the at least one mobile communication device (114) comprises the at least one screen (118) and the at least one camera (120).

13. The device (110) according to the preceding claim, wherein the at least one evaluation unit (124) is further configured to synchronize the displaying of the at least one illumination pattern (128) with the capturing of the at least one image (168).

14. The device (110) according to any one of the preceding device claims, wherein the at least one evaluation unit (124) is further configured to have access to the at least one illumination pattern (128) as displayed by the at least one screen (118) or to a reference thereto, and wherein the at least one evaluation unit (124) is further configured to compare the at least one image (168) with the at least one illumination pattern (128).

15. The device (110) according to any one of the preceding device claims, wherein the at least one mobile communication device (114) further comprises at least one of:
- a distance meter, wherein the distance meter is configured to determine at least one distance (148);
- a gyro sensor, wherein the gyro sensor is configured to determine at least one orientation of the at least one mobile communication device (114) in space;
- a holding unit attached to the at least one mobile communication device (114), wherein the holding unit is configured to maintain a position of the at least one mobile communication device (114).
